# EUROPEAN PATENT SPECIFICATION

(11) **EP 1 549 649 B1**
(45) Date of publication and mention of the grant of the patent: **26.07.2006**
(21) Application number: 03798307.9
(22) Date of filing: 18.09.2003
(51) Int. Cl.: C07D 471/04, A61K 31/415, A61P 25/00

(54) **PROCESS FOR PREPARING HALOALKYL PYRIMIDINES**
VERFAHREN ZUR HERSTELLUNG VON HALOGENALKYLPYRIMIDINEN
PROCEDE DE PREPARATION D'HALOALKYLE PYRIMIDINES

(30) Priority: 30.09.2002 US 415130 P
(43) Date of publication of application: 06.07.2005
(73) Proprietor: Pfizer Products Inc., Groton, Connecticut 06340 (US)
(72) Inventor: CARON, Stephane, Eastern Point Road, Groton, CT 06340 (US); DO, Nga My, Eastern Point Road, Groton, CT 06340 (US); MCDERMOTT, Ruth E., Eastern Point Road, Groton, CT 06340 (US); SINGER, Robert Alan, Eastern Point Road, Groton, CT 06340 (US)
(74) Representative: Bridle, Andrew Barry
(86) International application number: PCT/IB2003/004100
(87) International publication number: WO 2004/029052

(56) References cited:
- WO-A-02/50062

## Description

### Field of the Invention

This invention relates to a process for producing haloalkyl pyrimidines as intermediates in the production of benzimidazole and/or pyridylimidazole derivatives having high selectivity and/or high affinity to the benzodiazepine site of GABA_{A} receptors.

### Background

Various benzimidazole and pyridylimidazole derivatives that bind to the benzodiazepin site of GABA_{A} receptors, including human GABA_{A} receptors, are described in International Publication No. WO02/50062 A2. Various processes for producing these compounds are discussed in the publication, such as Scheme I which follows:

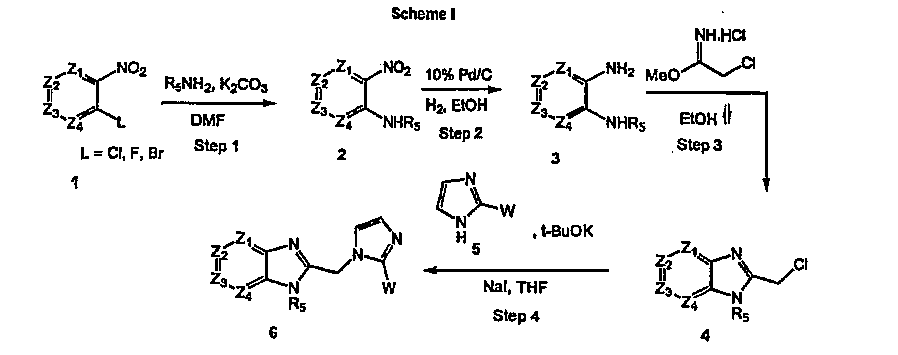

Scheme I illustrates a route to selected compounds of Formula **6** via coupling of chloromethyl compounds 4 and aryl imidazoles 5. In Step 1, aryl and heteroaryl halides of formula **1** are reacted with appropriate amines in the presence of base to obtain amino adducts of formula **2.** In Step 2, reduction of the nitro group in compounds of formula **2** yields diamines 3. In Step 3, diamines of formula **3** are reacted with 2-chloro-acetimidic acid methyl ester hydrochloride or a similar electrophile such as 2-chloro-1, 1, 1-trimethoxy-ethane or chloroacetic acid anhydride. In Step 4, chloromethyl compounds of formula **4** are reacted with aryl and heteroaryl imidazoles of formula **5** in the presence of base and solvent to obtain compounds of formula **6.** Depending on the particular nature of 5, a stronger or weaker base may be selected to facilitate the reaction in Step 4.

Several other schemes in the WO Publication, such as schemes 5, 7 and 8, utilize the chloromethyl compounds of formula 4 as an intermediate in processes for producing a wide range of benzimidazole and/or prridylimidazole compounds, useful in accordance with the description in the specification.

While a process for producing the intermediate haloalkyl pyrimidine of formula 4 is described in accordance with the process of Scheme I, the selected N-oxide reactant, and intermediate N-oxide compounds, together with the reaction conditions renders the process inefficient, costly and difficult for scale up to useful production volumes. The intermediate N-oxide is relatively unstable and this detrimentally affects yields. The N-oxide compound (1) is also not commercially available, requiring initial processing before the process of Scheme 1 can begin. Consequently, alternative methods for producing such an intermediate, at lower cost, with better ease in manufacture and with improved productivity, continue to be sought.

### Summary of Invention

The invention is a process for preparing a compound of formula A, 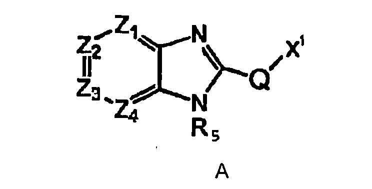 Where:
Z₁ is nitrogen or CR₁;
Z₂ is nitrogen or CR₂;
Z₃ is nitrogen or CR₃;
Z₄ is nitrogen or CR₄;
provided that no more than two of Z₁, Z₂, Z₃, and Z₄ are nitrogen;
R₁, R₂, R₃, and R₄ are independently selected from
   i) hydrogen or halogen,
   ii) alkyl, alkoxy, cycloalkyl, alkenyl, alkynyl, (cycloalkyl) alkyl, -N(R₁₀) (R₁₁), (R₁₀) (R₁₁) N-alkyl, (heterocycloalkyl) alkyl, heterocycloalkyl, aryl, and heteroaryl, each of which is optionally substituted with 1, 2, 3, or 4 of R₂₀, wherein R₁₀ and R₁₁ are independently selected from the group consisting of alkyl, alkenyl, alkynyl, alkoxy, cycloalkyl, (cycloalkyl) alkyl, aryl, arylalkyl; and
   iii) a group of the formula: 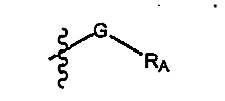
   where G is a bond, alkyl, -O-, and
R_{A} is a saturated, partially unsaturated, or aromatic substituted or unsubstituted carbocycle,
consisting of 1 ring or 2 fused, pendant, or spiro rings, each ring containing 0, 1, or 2 heteroatoms independently chosen from N, S, and O, said saturated, partially unsaturated, or aromatic carbocycle which is optionally substituted with 1, 2, 3, or 4 of R₂₀, and
R₂₀ is independently selected at each occurrence from the group consisting of: halogen; cyano; alkyl; alkoxy optionally substituted with dialkylamino; cycloalkyl; cycloalkylalkyl; cycloalkylalkoxy; alkenyl; alkynyl; dialkylamino; dialkylaminoalkyl;
R₅ is hydrogen; or
R₅ is alkyl, cycloalkyl, or (cycloalkyl)alkyl, each of which may optionally contain one or more double or triple bonds, and each of which is optionally substituted with 1, 2, or 3 of R_{30;}
R₃₀ is independently selected at each occurrence from alkyl, alkoxy optionally substituted with dialkylamino, cycloalkyl, cycloalkylalkyl, cycloalkylalkoxy, heterocycloalkyl, alkenyl, alkynyl, dialkylamino, aminoalkyl, and dialkylaminoalkyl;
X¹ is halogen;
Q is -CH(R₆), where R₆ independently represents hydrogen, or (C₁-C₆) alkyl, the process comprising;
reacting a diamino compound with an acylating agent;
reacting the resultant compound or a salt thereof with a reducing agent;
reacting the reduced resulting compound or a salt thereof with a haloalkylanoic acid anhydride. Alternately, the reduced resultant compound is acylated with the haloalkylanoic acid anhydride and cyclized in a solvent in the presence of an acid to provide the compound of formula A.

In another embodiment of the invention, the process comprises preparing a compound of formula A by reacting a diamino compound with a protecting group, and forming a subsequent imine by reaction with an aldehyde, then reducing the imine by treating with a reducing agent and then reacting with haloalkylanoic acid anhydride.

The process is preferably used for preparing a compound of Formula A wherein:
Z₁ is CR₁, Z₂ is CR₂, Z₃ is N, Z₄ is CR₄,
R₁, R₂, and R₄ are independently selected from hydrogen, halogen, cyano, amino, (C₁ -C₆) alkyl, (C₁ -C₆) alkoxy, (C₃ -C₈) cycloalkyl, (C₃ -C₈) cycloalkyl (C₁ -C₆) alkyl, di(C₁ -C₆) alkylamino, di(C₁ -C₆) alkylamino (C₁ -C₆) alkyl;
R₅ represents (C₁ -C₆) alkyl; and,
Q represents -CH₂.

The process according to the above is more preferably used to produce a compound of formula A wherein R₁ and R₄ are hydrogen, or wherein R₅ is ethyl or n-propyl, or wherein R₂ is chosen from:
i) hydrogen, halogen, cyano or
ii) C₁-C₆ alkyl, C₁-C₆ alkoxy, C₃-C₈ cycloalkyl, C₂-C₆ alkenyl, C₂-C₆ alkynyl, (C₃₋C₈ cycloalkyl) C₁-C₄ alkyl, -N(R₁₀)(R₁₁), and (R₁₀)(R₁₁)N(C₁-C₆) alkyl.

By utilizing a diamino compound as the initial reactant, the N-oxide instability is avoided and the processing conditions significanty improved, providing improved yields, higher productivity, at lower cost, as the diamino compounds are generally commercially available.

### Detailed Description of Invention

The invention is a process for producing haloalkyl pyrimidines of formula A, and salts thereof, as described above as intermediates in the production of benzimidazole and/or pyridylimidazole derivatives having high selectivity and/or high affinity to the benzodiazepine site of GABA_{A} receptors.

For this application, the term "salt" or "salts" refers to derivatives of the identified compound modified by making an acid or base salt thereof, as those skilled in organic synthesis would readily understand.

Also, the compounds referred to herein may have one or more asymmetric centers or planes, and that the compounds may be isolated in optically active or racemic forms, using various known procedures, such as chromatography or crystallization.

Various variables may occur more than one time in various compound formulas, and it should be understood that the definition at each occurance is independent of its definition at every other occurance.

The term "optionally substituted by one or more substituents" means that a group may be unsubstituted or have from 1 to the maximum number of substituents allowable without exceeding the valency of the atoms of the substituted group. Preferably such groups are unsubstituted or substituted with from 1 to 4 substituents, and more preferably such groups are either unsubstituted or substituted with from 1 to 3 substituents.

The term "alkyl" has its ordinary and accustomed meaning and specifically includes both branched and straight-chain aliphatic hydrocarbon groups, having the specified number of carbon atoms. Where there is reference to alkyl or other groups such as the C₁ -C₆ alkyl groups, the term "C₁ -C₆" means groups having from 1 to 6 carbon atoms. Alkyl groups of 2 or more carbon atoms may contain double or triple bonds. Examples of alkyl include, but are not limited to, methyl, ethyl, n-propyl, i-propyl, n-butyl, s-butyl, t-butyl, n-pentyl, and s-pentyl

The term "alkoxy" shall have its ordinary and accustomed meaning and specifically includes an alkyl group as defined above with the indicated number of carbon atoms attached through an oxygen bridge, such as methoxy, ethoxy, n-propoxy, i-propoxy, n-butoxy, 2-butoxy, t-butoxy, n-pentoxy, 2-pentoxy, 3-pentoxy, isopentoxy, neopentoxy, n-hexoxy, 2-hexoxy, 3-hexoxy, and 3-methylpentoxy, among others. As above, a term such as "C₁ -C₆" alkoxy indicates alkoxy groups having from 1 to 6 carbon atoms.

The term "alkenyl" has its ordinary and accustomed meaning and specifically includes hydrocarbon chains of either a straight or branched configuration comprising one or more unsaturated carbon-carbon bonds occuring in any stable point along the chain, such as ethenyl and propenyl, typically having from 2 to about 8 carbon atoms, more typically 2 to about 6 carbon atoms.

The term "alkynyl" has its ordinary and accustomed meaning and specifically includes hydrocarbon chains of either a straight or branched configuration comprising one or more triple carbon-carbon bonds which occur in any stable point along the chain, such as ethynyl and propynyl, typically having from 2 to about 8 carbon atoms, more typically 2 to about 6 carbon atoms.

The term "aryl" has its ordinary and accustomed meaning and specifically includes aromatic groups having 1 or more rings, the members of the aromatic ring or rings being carbon. The groups may be substituted, examples being optionally substituted phenyl and optionally substituted naphthyl.

The term "cycloalkyl" has its ordinary and accustomed meaning and specifically includes saturated ring groups, having the specified number of carbon atoms, such as cyclopropyl, cyclobutyl, cyclopentyl, or cyclohexyl, typically having 3 to about 8 ring members.

Where groups are identified together such as "(cycloalkyl) alkyl", the terms are defined as above, with the point of attachment identified, such as on the alkyl group in this case, which for example encompasses, but is not limited to, cyclopropylmethyl, cyclohexylmethyl, or cyclohexylmethyl.

The term "haloalkyl" has its ordinary and accustomed meaning and specifically includes both branched and straight-chain saturated aliphatic hydrocarbon groups having the specified number of carbon atoms, substituted with 1 or more halogen atoms. Examples of haloalkyl include, but are not limited to, trifluoromethyl, difluoromethyl, trichloromethyl, pentafluoroethyl, and pentachloroethyl.

The term "haloalkoxy" has its ordinary and accustomed meaning and specifically indicates a haloalkyl group as defined above with the indicated number of carbon atoms attached through an oxygen bridge. Examples of haloalkoxy groups include, but are not limited to, trifluoromethoxy and trichloromethoxy.

The term "heteroaryl" has its ordinary and accustomed meaning and specifically includes a stable 5-to 7-membered monocyclic or 7-to I0-membered bicyclic heterocyclic aromatic ring which consists of carbon atoms and from 1 to 4 heteroatoms independently selected from the group consisting of N, 0 and S, the total number of S and 0 atoms in the heteroaryl group preferably not being more than 1.

The heteroaryl groups may include, but are not limited to, pyrimidinyl, pyridyl, quinolinyl, benzothienyl, indolyl, pryidazinyl, pyazinyl, isoindolyl, isoquinolyl, quinazolinyl, quinoxalinyl, phthalazinyl, imidazolyl, isoxazolyl, pyrazolyl, oxazolyl, thienyl, thiazolyl, indolizinyl, indazolyl, benzothiazolyl, benzimidazolyl, benzofuranyl, benzoisoxolyl, dihydrobenzodioxinyl, furanyl, pyrrolyl, oxadiazolyl, thiadiazolyl, triazolyl, tetrazolyl, oxazolopyridinyl, imidazopyridinyl, isothiazolyl, naphthyridinyl, cinnolinyl, carbazolyl, beta-carbolinyl, isochromanyl, chromanonyl, chromanyl, tetrahydroisoquinolinyl, isoindolinyl, isobenzotetrahydrofuranyl, isobenzotetrahydrothienyl, isobenzothienyl, benzoxazolyl, pyridopyridinyl, benzotetrahydrofuranyl, benzotetrahydrothienyl, purinyl, benzodioxolyl, triazinyl, phenoxazinyl, phenothiazinyl, pteridinyl, benzothiazolyl, imidazopyridinyl, imidazothiazolyl, dihydrobenzisoxazinyl, benzisoxazinyl, benzoxazinyl, dihydrobenzisothiazinyl, benzopyranyl, benzothiopyranyl, coumarinyl, isocoumarinyl, chromanyl, tetrahydroquinolinyl, dihydroquinolinyl, dihydroquinolinonyl, dihydroisoquinolinonyl, dihydrocoumarinyl, II dihydroisocoumarinyl, isoindolinonyl, benzodioxanyl, benzoxazolinonyl, pyrrolyl N-oxide, pyrimidinyl N- oxide, pyridazinyl N-oxide, pyrazinyl N-oxide, quinolinyl N- oxide, indolyl N-oxide, indolinyl N-oxide, isoquinolyl N-oxide, quinazolinyl N-oxide, quinoxalinyl N-oxide, phthalazinyl N-oxide, imidazolyl N-oxide, isoxazolyl N-oxide, oxazolyl N-oxide, thiazolyl N-oxide, indolizinyl N-oxide, indazolyl N-oxide, benzothiazolyl N-oxide, benzimidazolyl N-oxide, pyrrolyl N-oxide, oxadiazolyl N-oxide, thiadiazolyl N-oxide, triazolyl N-oxide, tetrazolyl N-oxid-, benzothiopyranyl S-oxide, and benzothiopyranyl S,S-dioxide, preferrably imidazolyl, pyrrolyl, pyridyl, thiazolyl, pyrazolyl, thiazolyl, isoxazolyl, triazolyl, tetrazolyl, oxadiazolyl, pyrimidinyl, and oxazolyl.

The term "heterocycloalkyl" has its ordinary and accustomed meaning and specifically includes saturated ring groups having at least 1 heteroatom, typically having 3 to 8 ring atoms, preferably 5 to 7 ring atoms, the heteroatoms selected from N, S, and 0 with remaining ring atoms being carbon, such as morpholinyl, piperidinyl, piperazinyl, thiomorpholinyl, and pyrrolidinyl.

The term "monocyclic or bicyclic ring" refers to saturated, partially unsaturated, or aromatic rings or ring systems, other than those containing N-oxide, which optionally contain from 1 to 4 heteroatoms independently chosen from N, S, and 0 with remaining ring members being carbon, such as saturated and partially unsaturated rings or ring systems.

The term "oxo" has its ordinary and accustomed meaning and specifically means a carbonyl group.

The process of the invention is used to produce a compound of formula A or a salt thereof, 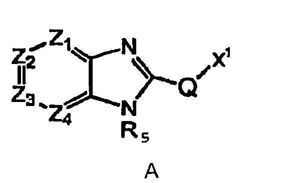 Where:
Z₁ is nitrogen or CR₁;
Z₂ is nitrogen or CR₂;
Z₃ is nitrogen or CR₃;
Z₄ is nitrogen or CR₄;
Provided, that no more than two of Z₁, Z₂, Z₃, and Z₄ are nitrogen;
R₁, R₂, R₃, and R₄ are independently selected from
   i) hydrogen or halogen,
   ii) alkyl, alkoxy, cycloalkyl, alkenyl, alkynyl, (cycloalkyl) alkyl, -N(R₁₀₎ (R₁₁), (R₁₀)(R₁₁)Nalkyl, (heterocycloalkyl) alkyl, heterocycloalkyl, aryl, and heteroaryl, each of which is optionally substituted with 1, 2, 3, or 4 of R₂₀, wherein R₁₀ and R₁₁ are independently selected from the group consisting of alkyl, alkenyl, alkynyl, alkoxy, cycloalkyl, (cycloalkyl) alkyl, aryl, arylalkyl; and
   iii) a group of the formula: 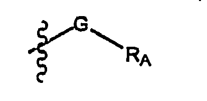
   where G is a bond, alkyl, -O-, and
R_{A} is a saturated, partially unsaturated, or aromatic carbocycle,
consisting of 1 ring or 2 fused, pendant, or spiro rings, each ring containing 0, 1, or 2 heteroatoms independently chosen from N, S, and O, said saturated, partially unsaturated, or aromatic carbocycle is optionally substituted with 1, 2, 3, or 4 of R₂₀, and
R₂₀ is independently selected at each occurrence from the group consisting of: halogen; cyano; alkyl; alkoxy optionally substituted with dialkylamino; cycloalkyl; cycloalkylalkyl; cycloalkylalkoxy; alkenyl; alkynyl; dialkylamino; and dialkylaminoalkyl;
R₅ represents hydrogen; or
R₅ represents alkyl, cycloalkyl, or (cycloalkyl) alkyl, each of which may contain one or more double or triple bonds, and each of which is optionally substituted with 1, 2, or 3 of R_{30;}
R₃₀ is independently selected at each occurrence from alkyl, alkoxy optionally substituted with dialkylamino, cycloalkyl, cycloalkylalkyl, cycloalkylalkoxy, heterocycloalkyl, alkenyl, alkynyl, dialkylamino, aminoalkyl, and dialkylaminoalkyl;
X¹ is halogen;
Q is -CH (R₆), where R₆ independently represents hydrogen, or C₁-C₆ alkyl.

Preferably, the process is used to produce a compound of formula A where
Z₁ is CR₁, Z₂ is CR₂, Z₃ is N, Z₄ is CR₄,
R₁, R₂, and R₄ are independently selected from hydrogen, halogen, (C₁ -C₆) alkyl, (C₁ - C₆) alkoxy, (C₃-C₈) cycloalkyl, (C₃-C₈) cycloalkyl (C₁-C₆) alkyl;
R₅ is (C₁-C₆) alkyl; and,
Q is -CH₂.

The inventive process proceeds in accordance with the following schemes 2, 2A and 3, which are presented for illustrative purposes only and the invention is not limited thereto.

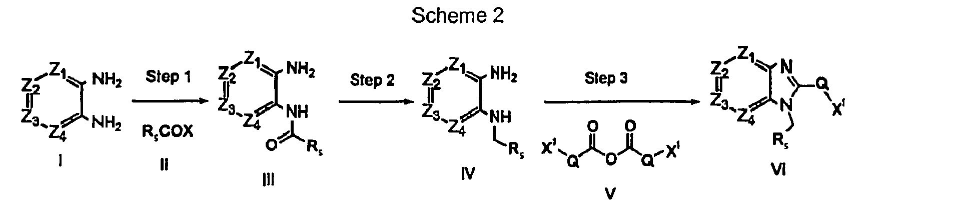

Step 1 of Scheme 2 is an acylation reaction. A diamino compound of formula I is reacted with an acylating agent which may be an acid halide or acid anhydride of formula II where X is halide (chloro, fluoro, bromo or iodo) or OCOR₅, though other acylation agents may be used. The reaction proceeds at a temperature between about -20° C and 60° C, preferably at room temperature, about 21° C, for a period of time between 1 to 48 hours, preferably 16 hours, to provide a product of formula III , which is produced either as the free base or as a salt.

By avoiding the N-oxide compounds and related processing steps of the prior scheme, yields are improved by at least 10 % and more likely, depending on the ultimate compound selected for production, can be improved by up to 70 %, while costs of the materials and processing equipment are reduced.

Step 2 of Scheme 2 is a reduction. The compound of formula III or a salt of a compound of formula III is reacted with a reducing agent in a solvent. The reducing agent is preferably a hydride reducing agent. Among the various reducing agents that may be used are lithium aluminum hydride, boron hydrides (borane), aluminum hydrides (alane), sodium aluminum hydride (Red-Al), and diisobutyl hydride (Dibal-H), among others. Preferably, lithium aluminum hydride or sodium aluminum hydride is used. Various solvents may be used, such as tetrahydrofuran, 1,4-dioxane, dimethoxyethane, diisopropyl ether, among others. Preferably, tetrahydrofuran is used. The reaction is undertaken at a temperature between about -78° C and 60° C, but preferably below about 25° C, for a period of time between about 1 to 30 hours, preferably about 16 hours, to provide the compound of formula IV.

In Step 3 of Scheme 2, the compound of formula IV is reacted in a solvent with a haloalkanoic acid anhydride (V), X¹ being hydrogen or halogen, at least one of X¹ being halogen, the halogen being independently selected from bromo, chloro, iodo or fluoro, Q being -CH(R₆), where R₆ independently represents hydrogen, or C₁-C₆ alkyl. Thus, various haloalkanoic acid anhydrides may be used. Examples may include but are not limited to chloro acetic acid anhydride, fluorohexanoic acid anhydride, difluoroacetic acid anhydride, chloropropanoic acid anhydride, etc. The solvent may be selected from, but is not limited to, ethyl acetate, isopropyl acetate, tetrahydrofuran, or toluene. Preferably, ethyl acetate is used. The reaction is conducted at a temperature between about 0° C and 50° C, preferably at room temperature, for a period of time between about 10 minutes and 24 hours, preferably 16 hours, to provide the intermediate haloalkyl pyrimidine compound of formula VI or a salt thereof.

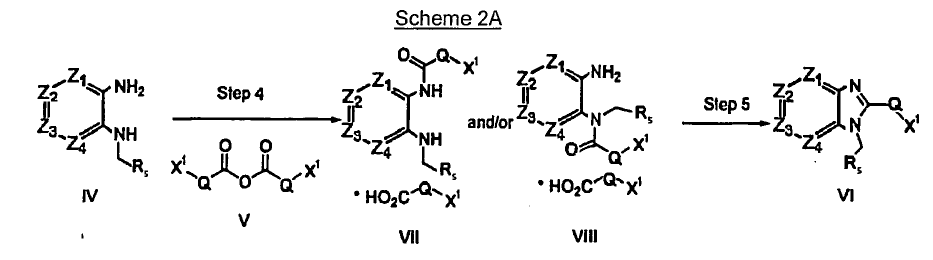

Alternatively, Step 3 of Scheme 2 is performed in two separate steps. Step 4 of Scheme 2A shows that the haloalkanoic acid salt of compound VII or VIII is generated by acylation of a compound of formula IV with the haloalkanoic acid anhydride V and cyclized in Step 5 of Scheme 2A. This is conducted in a solvent selected from but not limited to alcohols and acetates, such as isopropyl alcohol, methanol, ethanol, ethyl acetate, isopropyl acetate, or, tetrahydrofuran. Peferably, isopropyl alcohol is used and the reaction conducted in the presence of an acid such as hydrochloric acid, hydrobromic acid, or sulfuric acid. Preferably, hydrochloric acid is used. Following scheme 2a, the intermediate haloalkyl pyrimidine compound of formula VI or a salt thereof is produced.

Another alternative process of the present invention utilizes a protecting group, as illustrated in Scheme 3, for preparing the intermediate haloalkyl pyrimidine compounds of formula VI. The protecting group protects an amine group to prevent formation of various side chains during one or more steps in the synthesis of the coumpounds of the invention, after which it is removed. The use of such a protective group, and the conditions for attachment and removal would be understood by those skilled in organic synthesis. Generally, various carbonyl compounds, such as Boc (tert-butoxycarbonyl), Fmoc (9-fluorenylmethoxycarbonyl), related compounds and derivatives thereof may be used to protect a reactive amine group in preparation for cyclizing to produce the compound of formula VI.

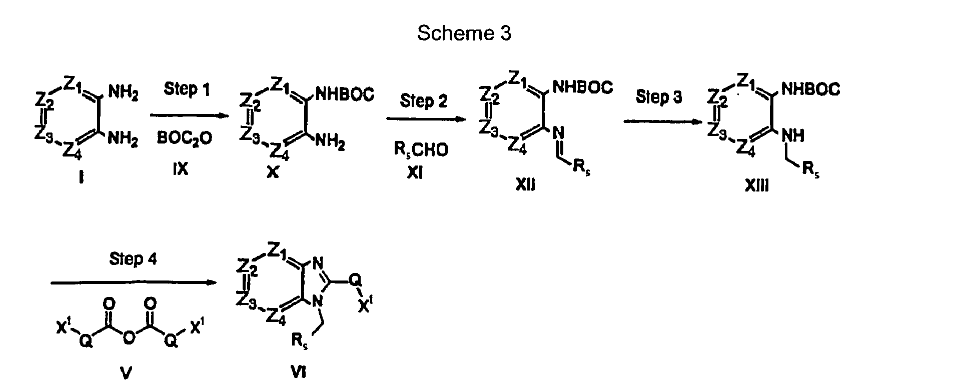

In step 1 of Scheme 3, a carbamate is formed by reacting the diamino compound of formula I, with a protecting group compound such as di-*tert*-butyl dicarbonate (IX) in a solvent. Various solvents could be used, with one solvent being dichloromethane. The reaction is conducted at a temperature between -20°C and room temperature, preferably at room temperature, to provide a compound of formula X.

Step 2 of Scheme 3 is an imine formation. The compound of formula X is reacted with an aldehyde of formula XI in a solvent, with R₅ as defined above. Again, the solvent may be selected from but is not limited to the alcohols and acetates, such as isopropyl alcohol, methanol, ethanol, ethyl acetate, isopropyl acetate, or, tetrahydrofuran. Preferably, ethanol is used and the reaction conducted at a temperature between -50°C and 60°C, preferably between 0°C and room temperature, to provide the imine of formula XII.

Step 3 of Scheme 3 is the reduction of the imine by reaction in a solvent with the reducing agent of formula V, discussed above, which may be for example sodium borohydride, sodium triacetoxy borohydride, sodium cyanoborohydride, lithium aluminum hydride, etc, with sodium borohydride preferred. The solvent is selected from but not limited to the alcohols and acetates, such as isopropyl alcohol, methanol, ethanol, ethyl acetate, isopropyl acetate or, tetrahydrofuran. Preferably, ethanol is used, and the reaction conducted at a temperature between -20°C and 60°C, preferably 0°C, producing the compound of formula XIII.

Alternatively, Steps 2 and 3 of Scheme 3 are combined and the reducing agent added to the reaction mixture upon completion of the imine formation to provide a compound of formula XIII without isolation of the imine of formula XII.

In Step 4 of Scheme 3, the compound of formula XII is reacted with the haloalkanoic acid anhydride (V) in a solvent, which may be selected from dichloromethane, ethyl acetate, isopropyl acetate, tetrahydrofuran, or toluene, among others. Preferably, dichloromethane is used and the reaction conducted at a temperature between 0°C and 80°C, preferably 30°C, in the presence of an acid, which may be selected from but is not limited to acids such as trifluoroacetic acid, sulfuric acid, hydrochloric acid, methane sulfonic acid, toluene sulfonic acid, etc. Preferably, trifluoroacetic acid is used. The reaction rate may vary depending on the reactants chosen, and can take from 1 hour to 7 days. In one example using chloroacetic acid anhydride, the reaction may take up to about 3 days, providing the intermediate haloalkyl pyrimidine compound of formula VI as the free base or as a salt thereof.

In an example of the process of the invention, using scheme 2, formula 1 is diaminopyridine, Q is CH₂ and X¹ is chlorine, yielding the following:

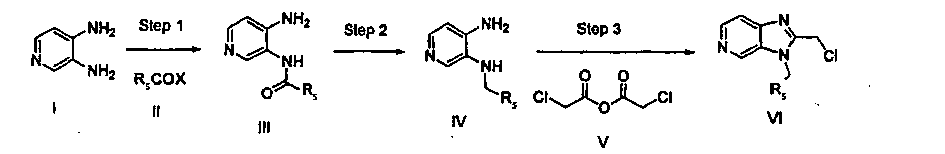

In the alternative process of scheme 2a, using the protective group for the same example, the following results:

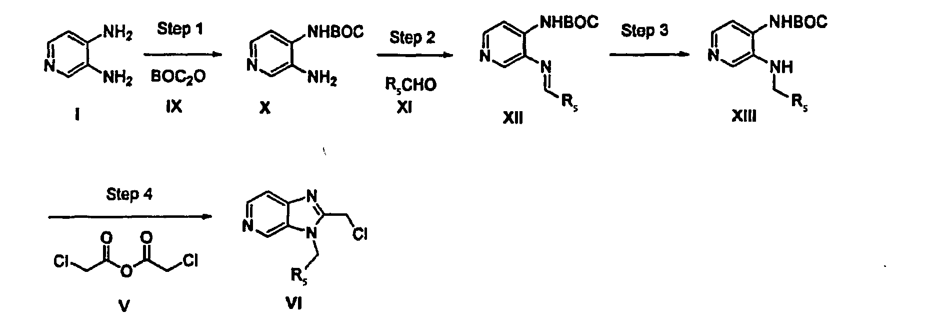

In the alternative process of scheme 3, using the same parameters, the following results:

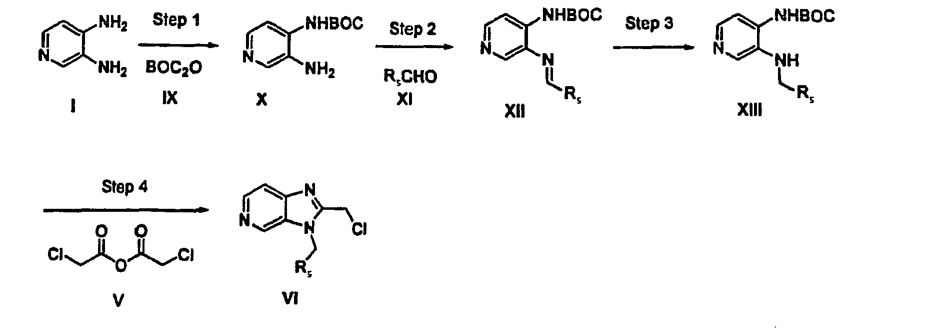

A second example, in accordance with scheme 2, and the preferences described above would proceed as follows, where R₅ is methyl:

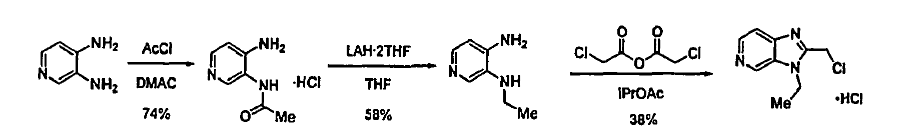

The same example, using the protective group process of scheme 2a would proceed as follows:

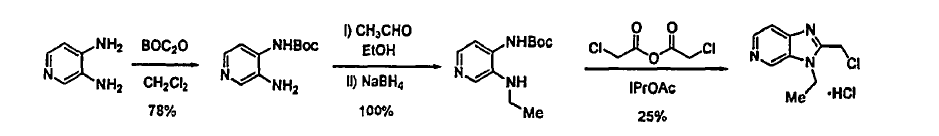

In the same example using the process of scheme 3, where R₅ is methyl, the following results:

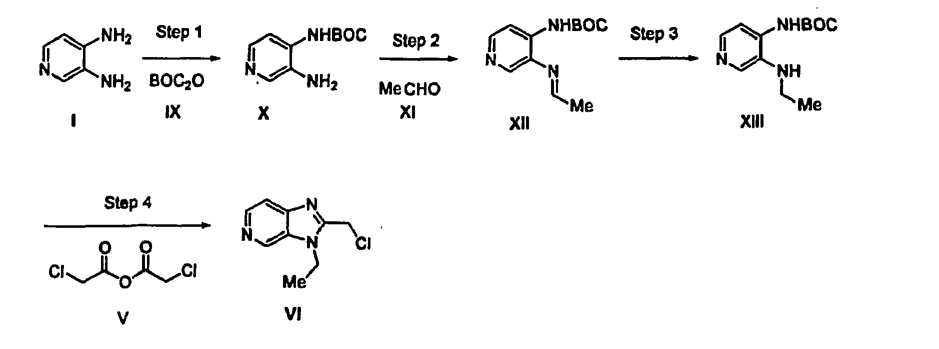

The following are further illustrative examples using the process of the invention, though the invention is not limited to the descriptions therein.

### EXAMPLES

### Example 1

### N-(4-Amino-pyridin-3-yl)-acetamide hydrochloride

To a solution of 3,4-diaminopyridine (10.53 g) in dimethyl acetamide (100 mL) was added slowly acetyl chloride (6.9 mL) keeping the temperature below 22° C. The reaction was stirred at room temperature for 16 hours whereupon cream solids had precipitated. The solids were filtered, washed with CH₂Cl₂ (2x50 mL), and dried under vacuum to give *N*-(4-amino-pyridin-3-yl)-acetamide hydrochloride (13.803g, 76%). M.p. =232-234°C decomp. ¹H NMR (400 MHz, *d*₆-DMSO): δ13.56 (s,1), 10.05 (s, 1), 8.52 (s, 1), 7.99 (d, 2, *J*=6.6 Hz), 6.9 (d, 1, *J*=6.6 Hz), 2.11 (s, 3). ¹³C NMR (100 MHz, *d*₆-DMSO) δ24.01, 109.88, 120.83, 134.53, 137.09, 154.26. IR 3353, 3187, 2950, 2836, 1651, 1563,1507, 1372, 1268, 1029, 817, 668, 577 cm⁻¹. Analysis calculated for C₇H₁₀CIN₃O: C, 44.81; H, 5.37; N, 22.40. Found: C, 44.80; H, 5.35; N, 22.18.

### Example 2

### N-3-Ethyl-pyridine-3,4-diamine

To a slurry of *N*-(4-amino-pyridin-3-yl)-acetamide hydrochloride (16.27 g) in THF (165 mL) under N₂ was added slowly, via an addition funnel, a 1.0 M solution of lithium aluminum hydride in THF (260 mL) while maintaining an internal temperature below 25° C. The resulting reaction mixture was stirred at room temperature for 16 hours. The resulting reaction mixture was cooled to 0°C and was quenched by addition of solid Na₂SO₄˙10H₂O (50g). The resulting mixture was warmed to room temperature and stirred for 2.5 hours. The reaction mixture was filtered through Celite and washed with ethyl acetate (2x50 mL). The filtrate was concentrated and crystallized from toluene to give N-3-Ethyl-pyridine-3,4-diamine (7.10 g, 60%). M.p. = 119-121°C. ¹H NMR (400 MHz, *d*₆-DMSO): δ7.49 (d, 2, J = 5.0), 6.37 (d, 1, *J* = 5.0), 5.38 (s, 1), 4.34 (t, I, *J* = 5.2), 3.34 (s, 1), 3.01 (qd, 2, *J* = 7.0 Hz, 5.4 Hz), 1.16 (t, 3, *J* =7.0 Hz). ¹³C NMR (100 MHZ, *d*₆-DMSO) δ15.16, 38.42, 108.41, 131.54, 135.96, 139.98, 142.28 cm⁻¹. Analysis calculated for C₇H₁₁N₃: C, 61.29: H, 8.08; N, 30.63. Found: C, 60.99; H, 8.05; N, 30.84.

### Example 3

### 2-Chloromethyl-3-ethyl-3H-imidazo[4,5-c]pyridine hydrochloride

To a solution of chloracetic anhydride (10.30 g) in ethyl acetate (40 mL) was added in one portion 3,4-diaminopyridine (IV) (2.01 g). After approximately 10 minutes, bright yellow solids had precipitated. The slurry was stirred at room temperature under N₂ for 16 hours. The reaction slurry was poured into 6 *N* NaOH (mL). The layers were separated and the organic layer was washed again with 1 *N* NaOH. The combined aqueous layers were back extracted with additional ethyl acetate (20mL). The combined organic phases were then washed with brine, dried over Na₂SO₄, and filtered. Concentrated hydrochloric acid (2 mL) was added and the filtrate was diluted with isopropanol (30 mL). All solvents were removed in vacuo. The resulting yellow soft solid was recrystallized from isopropanol to give 2-chloromethyl-3-ethyl 3*H*-imidazo[4,5-c]pyridine (2.02 g, 59%). Mp=218-220°C decomp. ¹H NMR (400 MHz, *d*_{*6*}-DMSO): δ9.64 (s, 1), 8.59 (d, 1, *J*=6.6), 8.21 (d, 1, J=6.6) 5.24 (s, 1), 4.56 (q, 2, *J*=7.2), 1.41 (t, 3, *J*=7.2). ¹³C NMR (100 MHz, *d*_{*6*}-DMSO) δ15.84, 36.34, 41.01, 117.36, 129.58, 133.20, 133.89, 151.66, 160.25. IR 3046, 2966, 2511, 1640, 1461, 1318, 848 cm⁻¹. Analysis calculated for C₉H₁₁Cl₂N₃: C, 46.57; H, 4.77; N, 18.10. Found: C, 46.79; H, 4.71; N, 17.93.

### Example 4

### (3-Amino-pyridin-4-yl)-carbamic acid tert-butyl ester

To a suspension of 3, 4-diaminopyridine (8.35g, 75 mmol) in dichloromethane (75 mL) was added dropwise di-*tert*-butyl dicarbonate (X) (16.73g, mmol) in dichloromethane. The reaction was allowed to stir at room temperature overnight. 1*N* Hydrochloric acid (86.2 mL) was added dropwise and the organic layer was separated. The aqueous layer was extracted with dichloromethane (75 mL) and the organic extracts were discarded. To the aqueous layer was added dichloromethane (75 mL). The mixture was stirred and potassium carbonate (8.25g) was added. The resulting pH of the aqueous layer was 8-9. The layers were separated and the aqueous layer was extracted with dichloromethane (2 x 75 mL). The organic extracts were combined, dried over sodium sulfate, filtered and concentrated. The product was crystallized from methyl tert-butyl ether and hexanes at 0° C to provide (3-amino-pyridin-4-yl)-carbamic acid tert-butyl ester as a light yellow solid (12.24g, 78%). Mp=124-126°C. ¹H NMR (400 MHz, *d*_{*6*}-DMSO) δ1.46 (s, 9), 5.08 (bs, 2), 7.50 (d, 1, *J*=5.2), 7.68 (d, 1, *J*=5.4) 7.91 (s, 1), 8.61 (s, 1). ¹³C NMR (100 MHz, *d*_{*6*}-DMSO) δ28.70, 80.47, 115.21, 131.28, 135.27, 138.41, 138.88, 153.37. IR 2978, 1716, 1588, 1515, 1249, 1154 cm⁻¹. Analysis calculated for C₁₀H₁₅N₃O₂: C, 57.40; H, 7.23; N, 20.08. Found: C, 57.50; H, 7.29; N, 20.06.

### Example 5

### (3-Ethylamino-pyridin-4-yl)-carbamic acid tert-butyl ester

To a solution of (3-amino-pyridin-4-y)-carbamic acid tert-butyl ester (5g, 23.89mmol) in ethanol (119 mL) at 0°C was added dropwise acetaldehyde (3.35mL, 58.72 mmol). The mixture was allowed to warm at room temperature and stirred overnight. The mixture was cooled to 0°C and sodium borohydride (2.26g, 59.74 mmol) was added in three portions keeping the temperature below 5° C. The reaction was allowed to warm to room temperature and was stirred for 10 hours. The mixture was cooled to 0°C and water (approx.. 140 mL) was added dropwise keeping the temperature below 5°C. Dichloromethane (100 mL) was added to the mixture followed by a dropwise addition of 10% aqueous citric acid until the pH was neutral. The mixture was stirred for an additional 30 minutes and dichloromethane (100 mL) was added. The layers were separated and the aqueous layer was extracted with dichloromethane. The combined organic extracts were dried over sodium sulfate, filtered, and concentrated to give (3-ethylamino-pyridin-4-yl)-carbamic acid *tert*-butyl ester (5.94 g of crude material, 89% purity, 94% yield). A small portion of the material was purified by silica gel chromatography (25% MeOH/MTBE) for characterization purposes. ¹H NMR (400 MHz, *d*₆-DMSO) δ1.20 (t, 3, *J*=7.1), 1.46 (s, 9), 3.04-3.11 (m, 2), 5.15 (t, 1, *J*=4.5), 7.54 (d, 1, *J*=5.2), 7.76 (d, 1, *J*=5.2), 7.83 (s, 1), 8.67 (s, 1). ¹³C NMR (100 MHz, *d*₆-DMSO) δ 14.89, 28.69, 38.30, 80.55, 115.05, 131.71, 133.53, 135.27, 138.84, 153.42. IR 2976, 1734, 1591, 1512, 1242, 1156 cm⁻¹. Analysis calculated for C₁₂H₁₉N₃O₂: C, 60.74; H, 8.07; N, 17.71. Found: C, 60.85; H, 7.95; N, 17.61.

### Example 6

### 2-Chloromethyl-3-ethyl-3H-imidazo[4,5-c]pyridine hydrochloride

To a solution of chloracetic anhydride (9.80 g, 57.3 mmol) in dichloromethane (44 mL) was added (3-ethylamino-pyridin-4-yl)-carbamic acid tert-butyl ester as a crude solid from the previous step (3.39g, 14.3 mmol). To the clear bright yellow solution was added trifuoroacetic acid (0.21 mL, 2.7 mmol). The reaction was stirred at 30°C for 3 days, poured into a separatory funnel and washed with 5*N* NaOH (32.5 mL). The layers were separated and the organic extract was washed with 1*N* NaOH (22.5 mL) and brine (22.5 mL). The organic extract was diluted with isopropanol (22 mL) and isopropyl acetate (74 mL), and concentrated hydrochloric acid (2.2 mL) was added. The mixture was concentrated to an orange solid under reduced pressure and the product was crystallized from isopropyl alcohol to afford 2-chloromethyl-3-ethyl-3*H*-imidazo[4,5-c]pyridine hydrochloride (1.78 g, 53%). This product was identical by ¹H NMR as the product from example 3.

While preferred embodiments of the present invention have been shown and described, it will be understood by those skilled in the art that various changes or modifications can be made without varying from the scope of the present invention.

## Claims

1. A process for producing a compound of formula A, 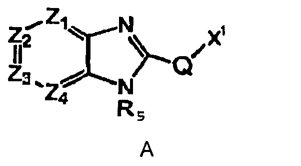 Where:
Z₁ is nitrogen or CR₁;
Z₂ is nitrogen or CR₂;
Z₃ is nitrogen or CR₃;
Z₄ is nitrogen or CR₄;
provided that no more than two of Z₁, Z₂, Z₃, and Z₄ are nitrogen;
R₁, R₂, R₃, and R₄ are independently selected from
i) hydrogen or halogen,
ii) alkyl, alkoxy, cycloalkyl, alkenyl, alkynyl, (cycloalkyl) alkyl, -N(R₁₀) (R₁₁), (R₁₀)(R₁₁)Nalkyl, (heterocycloalkyl) alkyl, heterocycloalkyl, aryl, and heteroaryl, each of which is optionally substituted with 1, 2, 3, or 4 of R₂₀, wherein R₁₀ and R₁₁ are independently selected from the group consisting of alkyl, alkenyl, alkynyl, alkoxy, cycloalkyl, (cycloalkyl) alkyl, aryl, arylalkyl; and
iii) a group of the formula: 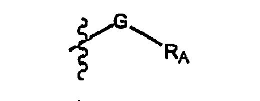
where G is a bond, alkyl, -O-, and
R_{A} is a saturated, partially unsaturated, or aromatic carbocycle,
consisting of 1 ring or 2 fused, pendant, or spiro rings, each ring containing 0, 1, or 2 heteroatoms independently chosen from N, S, and O, said saturated, partially unsaturated, or aromatic carbocycle is optionally substituted with 1, 2, 3, or 4 of R₂₀, and
R₂₀ is independently selected at each occurrence from the group consisting of: halogen; cyano; alkyl; alkoxy optionally substituted with dialkylamino; cycloalkyl; cycloalkylalkyl; cycloalkylalkoxy; alkenyl; alkynyl; dialkylamino; and dialkylaminoalkyl;
R₅ represents hydrogen; or
R₅ represents alkyl, cycloalkyl, or (cycloalkyl) alkyl, each of which may contain one or more double or triple bonds, and each of which is optionally substituted with 1, 2, or 3 of R_{30;}
R₃₀ is independently selected at each occurrence from alkyl, alkoxy optionally substituted with dialkylamino, cycloalkyl, cycloalkylalkyl, cycloalkylalkoxy, heterocycloalkyl, alkenyl, alkynyl, dialkylamino, aminoalkyl, and dialkylaminoalkyl;
X¹ is halogen;
Q is -CH(R₆), where R₆ independently represents hydrogen, or C₁-C₆alkyl, the process comprising;
reacting a diamino compound with an acylating agent;
reacting the resultant compound or a salt thereof with a reducing agent; and,
reacting the reduced resulting compound or a salt thereof with a haloalkanoic acid anhydride, to produce the compound of formula A or a salt thereof.

2. The process of claim 1 wherein the reduced resultant compound is acylated with the haloalkanoic acid anhydride and cyclized in a solvent in the presence of an acid to provide the compound of formula A.

3. The process of claim 1 wherein the acylating agent is R₅COX, and X is halide or -OCOR₅.

4. The process of claim 1 where the acylating step is conducted at a temperature about -20°C to about 60°C, for a period of time between 1 to 48 hours.

5. The process of claim 4 wherein the temperature is about 20° C, and the period is about 16 hours.

6. The process of claim 1 wherein the reducing agent is a hydride reducing agent selected from the group consisting of lithium aluminum hydride, boron hydrides, aluminum hydrides, sodium aluminum hydride, diisobutyl hydride and combinations thereof,

7. The process of claim 1 further comprising reacting the resultant compound with the reducing agent in a solvent, the solvent selected from the group consisting of tetrahydrofuran, 1,4-dioxane, dimethoxyethane, diisopropyl ether and combinations thereof.

8. The process of claim 1 comprising reacting the resultant compound with the reducing agent at a temperature about -78° C to about 60° C, for a period of between 1 and 30 hours.

9. The process of claim 1 further comprising reacting the reduced resultant compound with the haloalkanoic acid anhydride in a solvent, the solvent selected from the group consisting of ethyl acetate, isopropyl acetate, tetrahydrofuran, toluene and combinations thereof.

10. The process of claim 1 further comprising reacting the reduced resultant compound with the haloalkanoic acid anhydride at a temperature about 0° C to about 50° C, for a period of between 10 minutes and 24 hours.

11. The process of claim 1 wherein the diamino compound is diaminopyridine.

12. The process of claim 1 wherein the haloalkanoic acid anhydride has the following formula, 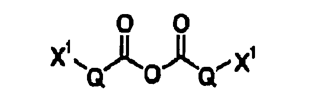 X¹ being hydrogen or halogen, at least one of X¹ being halogen, the halogen being independently selected from bromo, chloro iodo or fluoro, Q being -CH(R₆), where R₆ independently represents hydrogen, or C₁-C₆ alkyl.

13. The process of claim 1 wherein the haloalkanoic acid anhydride is chloroacetic anhydride.

14. The process of claim 1 wherein
Z₁ is CR₁, Z₂ is CR₂, Z₃ is N, Z₄ is CR₄,
R₁, R₂, and R₄ are independently selected from hydrogen, halogen, (C₁ -C₆) alkyl, (C₁ - C₆) alkoxy, (C₃ -C₈) cycloalkyl, (C₃-C₈) cycloalkyl (C₁ -C₆) alkyl;
R₅ is (C₁ -C₆) alkyl; and,
Q is -CH₂.

15. A process for producing a compound of the following formula A 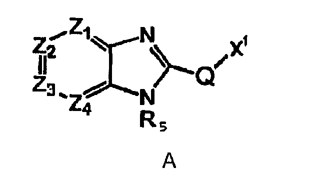 Where:
Z₁ is nitrogen or CR₁;
Z₂ is nitrogen or CR₂;
Z₃ is nitrogen or CR₃;
Z₄ is nitrogen or CR₄;
provided that no more than two of Z₁, Z₂, Z₃, and Z₄ are nitrogen;
R₁, R₂, R₃, and R₄ are independently selected from
i) hydrogen or halogen,
ii) alkyl, alkoxy, cycloalkyl, alkenyl, alkynyl, (cycloalkyl) alkyl, -N(R₁₀) (R₁₁), (R₁₀)(R₁₁)N-alkyl, (heterocycloalkyl) alkyl, heterocycloalkyl, aryl, and heteroaryl, each of which is optionally substituted with 1, 2, 3, or 4 of R₂₀, wherein R₁₀ and R₁₁ are independently selected from the group consisting of alkyl, alkenyl, alkynyl, alkoxy, cycloalkyl, (cycloalkyl) alkyl, aryl, arylalkyl; and
iii) a group of the formula: 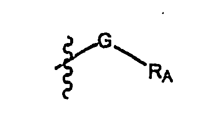
where G is a bond, alkyl, -O-, and
R_{A} is a saturated, partially unsaturated, or aromatic substituted or unsubstituted carbocycle,
consisting of 1 ring or 2 fused, pendant, or spiro rings, each ring containing 0, 1, or 2 heteroatoms independently chosen from N, S, and O, said saturated, partially unsaturated, or aromatic carbocycle is optionally substituted with 1, 2, 3, or 4 of R₂₀, and
R₂₀ is independently selected at each occurrence from the group consisting of: halogen; cyano; alkyl; alkoxy optionally substituted with dialkylamino; cycloalkyl; cycloalkylalkyl; cycloalkylalkoxy; alkenyl; alkynyl; dialkylamino; and dialkylaminoalkyl;
R₅ is hydrogen; or
R₅ represents alkyl, cycloalkyl, or (cycloalkyl) alkyl, each of which may contain one or more double or triple bonds, and each of which is optionally substituted with 1, 2, or 3 of R_{30;}
R₃₀ is independently selected at each occurrence from alkyl, alkoxy optionally substituted with dialkylamino, cycloalkyl, cycloalkylalkyl, cycloalkylalkoxy, heterocycloalkyl, alkenyl, alkynyl, dialkylamino, aminoalkyl, and dialkylaminoalkyl;
X¹ is halogen;
Q is -CH(R₆), where R₆ independently represents hydrogen, or C₁-C₆alkyl, the process comprising;
reacting a diamino compound with a compound containing a protective group;
reacting the resultant compound or a salt thereof with an aldehyde to form an imine compound or a salt thereof;
reacting the imine compound or a salt thereof with a reducing agent; and,
reacting the reduced imine compound or a salt thereof with a haloalkanoic acid anhydride, to produce the compound of formula A or a salt thereof.

## Patentansprüche

1. Verfahren zum Herstellen einer Verbindung der Formel A 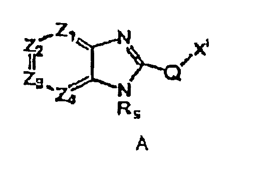 worin:
Z₁ Stickstoff oder CR₁ ist;
Z₂ Stickstoff oder CR₂ ist;
Z₃ Stickstoff oder CR₃ ist;
Z₄ Stickstoff oder CR₄ ist;
mit der Maßgabe, dass nicht mehr als zwei von Z₁, Z₂, Z₃ und Z₄ Stickstoff sind;
R₁, R₂, R₃ und R₄ unabhängig ausgewählt sind aus
i) Wasserstoff und Halogen,
ii) Alkyl, Alkoxy, Cycloalkyl, Alkenyl, Alkinyl, (Cycloalkyl) alkyl, -N(R₁₀₎(R₁₁), (R₁₀)(R₁₁)N-Alkyl, (Heterocycloalkyl)alkyl, Heterocycloalkyl, Aryl und Heteroaryl, wobei jedes davon gegebenenfalls substituiert ist mit 1, 2, 3 oder 4 von R₂₀, wobei R₁₀ und R₁₁ unabhängig ausgewählt sind aus der Gruppe, bestehend aus Alkyl, Alkenyl, Alkinyl, Alkoxy, Cycloalkyl, (Cycloalkyl)alkyl, Aryl, Arylalkyl; und
iii) einer Gruppe der Formel: 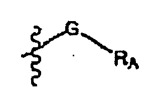
worin G eine Bindung, Alkyl, -O- ist, und
R_{A} ein gesättigter, partiell ungesättigter oder aromatischer Carbocyclus ist,
bestehend aus 1 Ring oder 2 kondensierten, herausstehenden oder Spiroringen, wobei jeder Ring 0, 1, oder 2 Heteroatome enthält, unabhängig ausgewählt aus N, S und O, wobei der gesättigte, partiell ungesättigte oder aromatische Carbocyclus gegebenenfalls mit 1, 2, 3 oder 4 von R₂₀ substituiert ist, und
R₂₀ bei jedem Vorkommen unabhängig ausgewählt ist aus der Gruppe, bestehend aus: Halogen, Cyano, Alkyl, Alkoxy, gegebenenfalls substituiert mit Dialkylamino, Cycloalkyl, Cycloalkylalkyl, Cycloalkylalkoxy, Alkenyl, Alkinyl, Dialkylamino und Dialkylaminoalkyl;
R₅ Wasserstoff darstellt; oder
R₅ Alkyl, Cycloalkyl oder (Cycloalkyl)alkyl darstellt, wobei jedes davon eine oder mehrere Doppel- oder Dreifachbindungen enthalten kann und wobei jedes davon gegebenenfalls substituiert ist mit 1, 2 oder 3 von R₃₀;
R₃₀ bei jedem Vorkommen unabhängig ausgewählt ist aus: Alkyl, Alkoxy, gegebenenfalls substituiert mit Dialkylamino, Cycloalkyl, Cycloalkylalkyl, Cycloalkylalkoxy, Heterocycloalkyl, Alkenyl, Alkinyl, Dialkylamino, Aminoalkyl und Dialkylaminoalkyl;
X¹ Halogen ist;
Q -CH(R₆) ist, wobei R₆ unabhängig Wasserstoff oder C₁-C₆₋Alkyl darstellt, wobei das Verfahren umfasst:
Reagieren lassen einer Diaminoverbindung mit einem Acylierungsmittel;
Reagieren lassen der resultierenden Verbindung oder eines Salzes davon mit einem Reduktionsmittel; und
Reagieren lassen der resultierenden reduzierten Verbindung oder eines Salzes davon mit einem Halogenalkansäureanhydrid, um die Verbindung A oder ein Salz davon herzustellen.

2. Verfahren nach Anspruch 1, wobei die resultierende reduzierte Verbindung mit dem Halogenalkansäureanhydrid acyliert wird und in einem Lösungsmittel in Gegenwart einer Säure cyclisiert wird, um eine Verbindung der Formel A bereit zu stellen.

3. Verfahren nach Anspruch 1, wobei das Acylierungsmittel R₅COX ist und X ein Halogenid oder -OCOR₅- ist.

4. Verfahren nach Anspruch 1, wobei der Acylierungschritt bei einer Temperatur von etwa -20 °C bis etwa 60 °C für eine Zeitdauer zwischen 1 bis 48 Stunden durchgeführt wird.

5. Verfahren nach Anspruch 4, wobei die Temperatur etwa 20 °C ist und die Dauer etwa 16 Stunden ist.

6. Verfahren nach Anspruch 1, wobei das Reduktionsmittel ein Hydridreduktionsmittel ist, ausgewählt aus der Gruppe, bestehend aus Lithiumaluminiumhydrid, Borhydride, Aluminiumhydride, Natriumaluminiumhydrid, Diisobutylhydrid und Kombinationen davon.

7. Verfahren nach Anspruch 1, weiterhin umfassend das Reagieren lassen der resultierenden Verbindung mit dem Reduktionsmittel in einem Lösungsmittel, wobei das Lösungsmittel ausgewählt ist aus der Gruppe, bestehend aus Tetrahydrofuran, 1,4-Dioxan, Dimethoxyethan, Diisopropylether und Kombinationen davon.

8. Verfahren nach Anspruch 1, umfassend das Reagieren lassen der resultierenden Verbindung mit dem Reduktionsmittel bei einer Temperatur von etwa -78 °C bis etwa 60 °C für eine Dauer von zwischen 1 und 30 Stunden.

9. Verfahren nach Anspruch 1, weiterhin umfassend das Reagieren lassen der resultierenden reduzierten Verbindung mit dem Halogenalkansäureanhydrid in einem Lösungsmittel, wobei das Lösungsmittel ausgewählt ist aus der Gruppe, bestehend aus Ethylacetat, Isopropylacetat, Tetrahydrofuran, Toluol und Kombinationen davon.

10. Verfahren nach Anspruch 1, weiterhin umfassend das Reagieren lassen der resultierenden reduzierten Verbindung mit dem Halogenalkansäureanhydrid bei einer Temperatur von etwa 0 °C bis etwa 50 °C für eine Dauer von zwischen 10 Minuten und 24 Stunden.

11. Verfahren nach Anspruch 1, wobei die Diaminoverbindung Diaminopyridin ist.

12. Verfahren nach Anspruch 1, wobei das Halogenalkansäureanhydrid die folgende Formel hat, 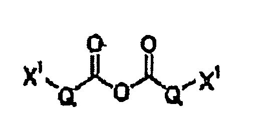 worin X¹ Wasserstoff oder Halogen ist, wobei mindestens eines von X¹ Halogen ist, wobei das Halogen unabhängig ausgewählt ist aus Brom, Chlor, Iod und Fluor, wobei Q -CH(R₆) ist, wobei R₆ unabhängig Wasserstoff oder C₁-C₆-Alkyl darstellt.

13. Verfahren nach Anspruch 1, wobei das Halogenalkansäureanhydrid Chloressigsäureanhydrid ist.

14. Verfahren nach Anspruch 1, wobei
Z₁ CR₁ ist, Z₂ CR₂ ist, Z₃ N ist, Z₄ CR₄ ist;
R₁, R₂ und R₄ unabhängig ausgewählt sind aus Wasserstoff, Halogen, (C₁-C₆)-Alkyl, (C₁-C₅)-Alkoxy, (C₃-C₈)-Cycloalkyl, (C₃₋C₈)- Cycloalkyl-(C₁-C₆)-alkyl;
R₅ (C₁-C₆)-Alkyl ist; und
Q -CH₂ ist.

15. Verfahren zum Herstellen einer Verbindung der folgenden Formel A 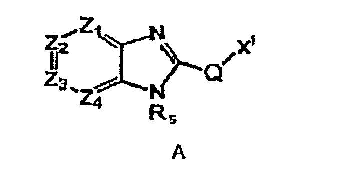 worin:
Z₁ Stickstoff oder CR₁ ist;
Z₂ Stickstoff oder CR₂ ist;
Z₃ Stickstoff oder CR₃ ist;
Z₄ Stickstoff oder CR₄ ist;
mit der Maßgabe, dass nicht mehr als zwei von Z₁, Z₂, Z₃ und Z₄ Stickstoff sind;
R₁, R₂, R₃ und R₄ unabhängig ausgewählt sind aus
i) Wasserstoff und Halogen,
ii) Alkyl, Alkoxy, Cycloalkyl, Alkenyl, Alkinyl, (Cycloalkyl)alkyl, -N(R₁₀)(R₁₁), (R₁₀)(R₁₁)N-Alkyl, (Heterocycloalkyl)alkyl, Heterocycloalkyl, Aryl und Heteroaryl, wobei jedes davon gegebenenfalls substituiert ist mit 1, 2, 3 oder 4 von R₂₀, wobei R₁₀ und R₁₁ unabhängig ausgewählt sind aus der Gruppe, bestehend aus Alkyl, Alkenyl, Alkinyl, Alkoxy, Cycloalkyl, (Cycloalkyl)alkyl, Aryl, Arylalkyl; und
iii) einer Gruppe der Formel: 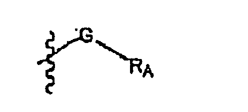
worin G eine Bindung, Alkyl, -O- ist, und
R_{A} ein gesättigter, partiell ungesättigter oder aromatischer substituierter oder unsubstituierter Carbocyclus ist,
bestehend aus 1 Ring oder 2 kondensierten, herausstehenden oder Spiroringen, wobei jeder Ring 0, 1, oder 2 Heteroatome enthält, unabhängig ausgewählt aus N, S und O, wobei der gesättigte, partiell ungesättigte oder aromatische Carbocyclus gegebenenfalls mit 1, 2, 3 oder 4 von R₂₀ substituiert ist, und
R₂₀ bei jedem Vorkommen unabhängig ausgewählt ist aus der Gruppe, bestehend aus: Halogen, Cyano, Alkyl, Alkoxy, gegebenenfalls substituiert mit Dialkylamino, Cycloalkyl, Cycloalkylalkyl, Cycloalkylalkoxy, Alkenyl, Alkinyl, Dialkylamino und Dialkylaminoalkyl;
R₅ Wasserstoff darstellt; oder
R₅ Alkyl, Cycloalkyl oder (Cycloalkyl)alkyl darstellt, wobei jedes davon eine oder mehrere Doppel- oder Dreifachbindungen enthalten kann und wobei jedes davon gegebenenfalls substituiert ist mit 1, 2 oder 3 von R₃₀;
R₃₀ bei jedem Vorkommen unabhängig ausgewählt ist aus Alkyl, Alkoxy, gegebenenfalls substituiert mit Dialkylamino, Cycloalkyl, Cycloalkylalkyl, Cycloalkylalkoxy, Heterocycloalkyl, Alkenyl, Alkinyl, Dialkylamino, Aminoalkyl und Dialkylaminoalkyl;
X¹ Halogen ist;
Q -CH(R₆) ist, wobei R₆ unabhängig Wasserstoff oder C₁-C₆₋Alkyl darstellt, wobei das Verfahren umfasst:
Reagieren lassen einer Diaminoverbindung, die eine Schutzgruppe enthält;
Reagieren lassen der resultierenden Verbindung oder eines Salzes davon mit einem Aldehyd, um eine Iminverbindung oder ein Salz davon zu bilden;
Reagieren lassen der Iminverbindung oder eines Salzes davon mit einem Reduktionsmittel; und
Reagieren lassen der reduzierten Iminverbindung oder eines Salzes davon mit einem Halogenalkansäureanhydrid, um die Verbindung A oder ein Salz davon herzustellen.

## Revendications

1. Procédé de production d'un composé de formule A, 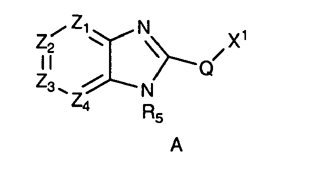 dans laquelle :
Z₁ représente l'azote ou CR₁ ;
Z₂ représente l'azote ou CR₂ ;
Z₃ représente l'azote ou CR₃ ;
Z₄ représente l'azote ou CR₄ ;
pour autant que pas plus de deux parmi Z₁, Z₂, Z₃ et
Z₄ représentent l'azote ;
R₁, R₂, R₃ et R₄ sont indépendamment sélectionnés parmi :
i) l'hydrogène ou un halogène,
ii) alkyle, alkoxy, cycloalkyle, alkényle, alkynyle, (cycloalkyl)alkyle, -N(R₁₀)(R₁₁), (R₁₀)(R₁₁)N-alkyle, (hétérocycloalkyl)alkyle, hétérocycloalkyle, aryle et hétéroaryle, dont chacun est facultativement substitué par 1, 2, 3 ou 4 R₂₀, où R₁₀ et R₁₁ sont indépendamment sélectionnés dans le groupe consistant en alkyle, alkényle, alkynyle, alkoxy, cycloalkyle, (cycloalkyl)alkyle, aryle, arylalkyle ; et
iii) un groupe de formule : 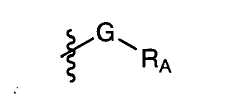
dans laquelle :
G représente une liaison, alkyle, -O-, et
R_{A} est un carbocycle saturé, partiellement insaturé ou aromatique consistant en un noyau ou en deux noyaux condensés, en pendentifs ou spiranniques, chaque noyau contenant 0, 1 ou 2 hétéroatomes indépendamment choisis parmi N, S et O, ledit carbocycle saturé partiellement insaturé ou aromatique étant facultativement substitué par 1, 2, 3 ou 4 R₂₀, et
R₂₀ est indépendamment sélectionné, à chaque apparition, dans le groupe consistant en halogéno, cyano, alkyle, alkoxy facultativement substitué par dialkylamino, cycloalkyle, cycloalkylalkyle, cycloalkylalkoxy, alkényle, alkynyle, dialkylamino, et dialkylaminoalkyle ;
R₅ représente l'hydrogène ; ou
R₅ représente alkyle, cycloalkyle, ou (cycloalkyl)alkyle, dont chacun peut contenir une ou plusieurs double- ou triple-liaisons, et dont chacun est facultativement substitué par 1, 2 ou 3 R₃₀ ;
R₃₀ est indépendamment sélectionné, à chaque apparition, parmi alkyle, alkoxy facultativement substitué par dialkylamino, cycloalkyle, cycloalkylalkyle, cycloalkylalkoxy, hétérocycloalkyle, alkényle, alkynyle, dialkylamino, aminoalkyle, et dialkylaminoalkyle ;
X₁ représente un halogène ;
Q représente -CH(R₆), où R₆ représente indépendamment l'hydrogène ou alkyle en C₁-C₆, le procédé comprenant ;
. la réaction d'un composé diamino avec un agent d'acylation ;
. la réaction du composé résultant, ou d'un sel dudit composé, avec un agent réducteur ; et
. la réaction du composé réduit résultant, ou d'un sel dudit composé, avec un anhydride d'acide halogénoalkanoïque pour produire le composé de formule A ou un sel dudit composé.

2. Procédé selon la revendication 1, dans lequel le composé réduit résultant est acylé avec l'anhydride d'acide halogénoalkanoïque et cyclisé dans un solvant en présence d'un acide pour fournir le composé de formule A.

3. Procédé selon la revendication 1, dans lequel l'agent d'acylation est R₅COX, et X est un halogénure ou -OCOR₅.

4. Procédé selon la revendication 1, dans lequel l'étape d'acylation est mise en oeuvre à une température comprise entre environ -20°C et environ 60°C, pendant une durée comprise entre 1 et 48 heures.

5. Procédé selon la revendication 4, dans lequel la température est d'environ 20°C et la durée est d'environ 16 heures.

6. Procédé selon la revendication 1, dans lequel l'agent réducteur est un agent réducteur hydrure sélectionné dans le groupe consistant en l'hydrure de lithium-aluminium, les hydrures de bore, les hydrures d'aluminium, l'hydrure de sodium-aluminium, l'hydrure de diisobutyle, et leurs combinaisons.

7. Procédé selon la revendication 1, comprenant en outre la réaction du composé résultant avec l'agent réducteur dans un solvant, le solvant étant sélectionné dans le groupe consistant en le tétrahydrofurane, le 1,4-dioxane, le diméthoxyéthane, l'éther diisopropylique, et leurs combinaisons.

8. Procédé selon la revendication 1, comprenant la réaction du composé résultant avec l'agent réducteur à une température comprise entre environ -78°C et environ 60°C, pendant une durée comprise entre 1 et 30 heures.

9. Procédé selon la revendication 1, comprenant en outre la réaction du composé réduit résultant avec l'anhydride d'acide halogénoalkanoïque dans un solvant, le solvant étant sélectionné dans le groupe consistant en l'acétate d'éthyle, l'acétate d'isopropyle, le tétrahydrofurane, le toluène, et leurs combinaisons.

10. Procédé selon la revendication 1, comprenant en outre la réaction du composé réduit résultant avec l'anhydride d'acide halogénoalkanoïque à une température comprise entre environ 0°C et environ 50°C, pendant une durée comprise entre 10 minutes et 24 heures.

11. Procédé selon la revendication 1, dans lequel le composé diamino est la diaminopyridine.

12. Procédé selon la revendication 1, dans lequel l'anhydride d'acide halogénoalkanoïque a la formule suivante : 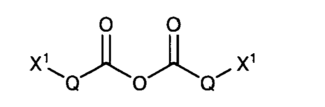 X¹ représentant l'hydrogène ou un halogène, l'un au moins des X¹ représentant un halogène, l'halogène étant indépendamment sélectionné parmi bromo, chloro, iodo ou fluoro, Q représentant -CH(R₆), où R₆ représente indépendamment l'hydrogène ou alkyle en C₁-C₆.

13. Procédé selon la revendication 1, dans lequel l'anhydride d'acide halogénoalkanoïque est l'anhydride chloroacétique.

14. Procédé selon la revendication 1, dans lequel :
Z₁ représente CR₁, Z₂ représente CR₂, Z₃ représente N,
R₄ représente CR₄ ;
R₁, R₂ et R₄ sont indépendamment sélectionnés parmi l'hydrogène, un halogène, alkyle en C₁-C₆, alkoxy en C₁-C₆, cycloalkyle en C₃-C₈, (cycloalkyle en C₃-C₈)alkyle en C₁-C₆ ;
R₅ représente alkyle en C₁-C₆ ; et
Q représente CH₂.

15. Procédé de production d'un composé de formule 1 suivante : 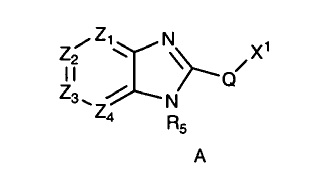 dans laquelle :
Z₁ représente l'azote ou CR₁ ;
Z₂ représente l'azote ou CR₂ ;
Z₃ représente l'azote ou CR₃ ;
Z₄ représente l'azote ou CR₄ ;
pour autant que pas plus de deux parmi Z₁, Z₂, Z₃ et
Z₄ représentent l'azote ;
R₁, R₂, R₃ et R₄ sont indépendamment sélectionnés parmi :
i) l'hydrogène ou un halogène,
ii) alkyle, alkoxy, cycloalkyle, alkényle, alkynyle, (cycloalkyl)alkyle, -N(R₁₀)(R₁₁), (R₁₀)(R₁₁)N-alkyle, (hétérocycloalkyl)alkyle, hétérocycloalkyle, aryle et hétéroaryle, dont chacun est facultativement substitué par 1, 2 , 3 ou 4 R₂₀, où R₁₀ et R₁₁ sont indépendamment sélectionnés dans le groupe consistant en alkyle, alkényle, alkynyle, alkoxy, cycloalkyle, (cycloalkyl)alkyle, aryle, arylalkyle ; et
iii) un groupe de formule : 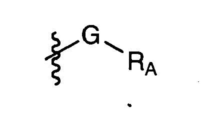
dans laquelle :
G représente une liaison, alkyle, -O-, et
R_{A} est un carbocycle saturé, partiellement insaturé ou aromatique, substitué ou non substitué, consistant en un noyau ou en deux noyaux condensés, en pendentifs ou spiranniques, chaque noyau contenant 0, 1 ou 2 hétéroatomes indépendamment choisis parmi N, S et O, ledit carbocycle saturé partiellement insaturé ou aromatique étant facultativement substitué par 1, 2, 3 ou 4 R₂₀, et
R₂₀ est indépendamment sélectionné, à chaque apparition, dans le groupe consistant en halogéno, cyano, alkyle, alkoxy facultativement substitué par dialkylamino, cycloalkyle, cycloalkylalkyle, cycloalkylalkoxy, alkényle, alkynyle, dialkylamino, et dialkylaminoalkyle ;
R₅ représente l'hydrogène ; ou
R₅ représente alkyle, cycloalkyle, ou (cycloalkyl)alkyle, dont chacun peut contenir une ou plusieurs double- ou triple-liaisons, et dont chacun est facultativement substitué par 1, 2 ou 3 R₃₀ ;
R₃₀ est indépendamment sélectionné, à chaque apparition, parmi alkyle, alkoxy facultativement substitué par dialkylamino, cycloalkyle, cycloalkylalkyle, cycloalkylalkoxy, hétérocycloalkyle, alkényle, alkynyle, dialkylamino, aminoalkyle, et dialkylaminoalkyle ;
X₁ représente un halogène ;
Q représente -CH(R₆), où R₆ représente indépendamment l'hydrogène ou alkyle en C₁-C₆, le procédé comprenant ;
. la réaction d'un composé diamino avec un composé contenant un groupe protecteur ;
. la réaction du composé résultant, ou d'un sel dudit composé, avec un aldéhyde pour former un composé imine ou un sel dudit composé ;
. la réaction du composé imine, ou d'un sel dudit composé, avec un agent réducteur ; et
. la réaction du composé imine réduit, ou d'un sel dudit composé, avec un anhydride d'acide halogénoalkanoïque, pour produire le composé de formule A ou un sel dudit composé.
